Europäisches Patentamt

European Patent Office

Office européen des brevets

(19)

(11) Publication number: **0 235 906**
**B1**

(12) **EUROPEAN PATENT SPECIFICATION**

(45) Date of publication of the patent specification:
26.09.90

(21) Application number: **87300589.6**

(22) Date of filing: **23.01.87**

(51) Int. Cl.⁵: **A61K 37/66**
**// A61K37:02**

(54) Method and composition for prophylaxis and treatment of viral infections.

(30) Priority: **24.01.86 US 822099**
**31.07.86 US 892531**

(43) Date of publication of application:
**09.09.87 Bulletin 87/37**

(45) Publication of the grant of the patent:
**26.09.90 Bulletin 90/39**

(84) Designated Contracting States:
**AT BE CH DE ES FR GB GR IT LI LU NL SE**

(56) References cited:
**EP-A- 0 128 009**
**EP-A- 0 131 789**
**EP-A- 0 170 843**
**WO-A-86/03751**
**DE-A- 3 227 262**

(73) Proprietor: **GENENTECH, INC., 460 Point San Bruno Boulevard, South San Francisco California 94080(US)**

(72) Inventor: **Wong, Grace Hing-Wah, 555 Grand Avenue Apt. 4, South San Francisco California 94080(US)**

(74) Representative: **Armitage, Ian Michael et al, MEWBURN ELLIS & CO. 2/3 Cursitor Street, London EC4A 1BQ(GB)**

## Description

This invention relates to the treatment of animals which have been virally infected or which are at risk for exposure to viral infection, particularly retroviruses. In particular, it is concerned with inducing in mammalian cells a state of resistance to viral infection and treating retrovirally infected humans.

This invention is especially concerned with the therapy of retroviral infections leading to immunological defects, more specifically those infections thought to be responsible for acquired immune deficiency syndrome (AIDS).

AIDS is a transmissible deficiency of cellular immunity characterized by opportunistic infections and certain malignancies, notably Pneumocystis carinii pneumonia and Kaposi's sarcoma, in patients without another recognized cause for contracting these rare diseases (1-3). AIDS is manifested by a profound lymphopenia, a generalized cutaneous anergy and markedly reduced proliferative response to mitogens, antigens and allogeneic cells, seeming to result from depletion of the OKT4+ T-lymphocyte subset (4). While humoral immunity is relatively unaffected, there is increasing evidence for a hyperactive B-cell proliferative response which may be related causally to the high incidence of B-lymphoma in AIDS patients (5,6). In addition to the fully developed syndrome, an epidemic of a related disease, AIDS-related complex (ARC), has appeared, characterized by generalized chronic lymphadenopathy. This syndrome shares many of the epidemiological features and immune abnormalities and often precedes the clinical manifestations of AIDS.

Recent evidence has implicated strongly a lymphocytotropic retrovirus as the primary aetiological agent of AIDS and the AIDS-related complex. Lymphadenopathy-associated virus (LAV) was isolated initially from cultured lymph-node T cells of patients with lymphadenopathy and AIDS as well as an AIDS patient and an asymptomatic sibling, both with haemophilia B (7-9). A similar virus, designated human T-lymphotrophic virus type III (HTLV-III), has been isolated from a large number of AIDS and ARC patient blood samples by co-cultivation with the permissive T-cell line H9 (10, 11). LAV and HTLV-III, as well as related retroviruses isolated recently from AIDS patients (12,13), share several important characteristics. Viral replication occurs in the OKT4+ T-lymphocyte population in vivo and in vitro and is associated with impaired cell proliferation and the appearance of cytopathic effects (8,10,14). The virus has a $Mg^{2+}$-dependent reverse transcriptase, exhibits a dense cylindrical core morphology similar to type D retroviruses (8,13,15) and is recognized by antibodies found in the sera of virtually all AIDS and ARC patients (8,13,16-21). HTLV-III and LAV now are believed to be strains of the same virus, which has been given the name human immunodeficiency virus (HIV).

Immune function in AIDS or ARC afflicted patients is severely compromised. However, the precise nature of the abnormalities induced by HIV continue to be under study. Of particular interest was the effect of HIV infection on the production of lymophokines by the immune cells of such patients as well as their responses to exogenously administered lymphokines.

Of 16 AIDS patients tested for T lymphocyte capacity to secrete macrophage-activating products, including gamma interferon, 14 failed to generate active lymphokines and 13-14 completely failed to secrete gamma interferon. Furthermore, macrophages from patients with AIDS showed enhanced antimicrobial activity when incubated in vitro with gamma interferon, thus raising the possibility that such cells may respond in vivo to exogenously administered gamma interferon (22, 24).

Lymphocytes from AIDS patients also have been reported to be deficient in their ability to product interleukin-2, a lymphokine having a role in the proliferation and differentiation of T lymphocytes and which stimulates gamma interferon production (23).

Finally, gamma interferon was known to exert a direct antiviral effect on vesicular stomatitis virus (VSV) infected cells and to potentiate the effect of a cytokine on VSV infected cells (25). This cytokine was tentatively identified by these authors as the polypeptide referred to herein as tumor necrosis factor-α. See also Eifel et al., "Cell Immun." 47: 197-203 (1979). Immunosupplementation therapy for AIDS therefore appeared to be a promising opportunity, and clinical studies with patients were instituted.

Despite the expectations for lymphokine therapy of AIDS patients, in vivo results have been disappointing. Contrary to the results encountered in in vitro experiments, a prolonged course of intravenous recombinant gamma interferon treatment appeared to inhibit rather than enhance monocyte respiratory burst function (26), and intravenous treatment of AIDS patients with varying doses of interleukin-2 or gamma interferon led to the conclusion that in vivo lymphokine therapy was not of significant value in the treatment of patients with AIDS (27). In fact, by the time of the invention herein, a school of thought had developed that the immune stimulation or reconstruction approach to AIDS therapy was actually "dangerous" because cell activation by lymphokines was though to contribute to virus spreading and T4 cell depletion, and thus would accelerate progression of the disease (28). In June, 1986, Yamamoto et al. (29) disclosed that human alpha and beta (but not gamma) interferons suppressed the invitro replication of HIV strains, but when exposure to the interferons ceased the viral production of infected cells was enhanced. This further suggested that immune therapy of AIDS would be counterproductive.

Finally, it has been suggested that lymphotoxin is produced by HIV-infected T cells by virally-induced transactivation of the T cells' endogenous lymphotoxin gene, thus leading to secretion of autotoxic quantities of lymphotoxin (35).

Interferons have been used to varying effect for the prophylaxis or treatment of chronic, acute and/or experimental infections by viruses such as vaccinia, rubella, herpes simplex, varicella-zoster, chicken pox, cytomegalovirus, adenovirus, ebola virus, rabies and hepatities B. Chronic cytomegalovirus infections have proven difficult to treat by interferons, even using much higher doses than were needed than for the prevention of CMV infections; these doses may involve undesirable side reactions such as neutropenia and suppression of weight gain. Accordingly, it would be desirable in patients with persistent or chronic viral infections to provide an interferon preparation that is more efficacious without inducing interferon side-effects.

Interferons also have been widely tested for their ability to prevent or treat the common cold (primarily rhinovirus infections). Most studies use intranasally administered daily doses ranging from $0.8 \times 10^6$ to $42.8 \times 10^6$ units (Finter et al., 1985, Interferon Vol. 4 pp. 186-187). However, it should be understood that the relationship between the specific activities reported by early workers and the International Standards now in use is unclear; the dosages employed when expressed in International Units could have been greater or lower. Intranasal administration generally was as an aerosol, although delivery by soaked pledget has been reported as more efficacious. Typically, the daily dosage is aliquoted and administered 1 to 3 times per day. Experience with recombinant interferons has shown that sprays delivering about $1 \times 10^6$ units/dose (at 0.1 ml/nostril) were effective in conferring protection whereas doses at one-tenth or one-one hundredth of this had no detectable effect (Finter et al., op cit, p. 188). However, doses of about $1 \times 10^6$ units of interferon have been associated with annoying, mild nasal irritation and greater doses are markedly irritating (Finter et al.Id.).

Interferons also have been administered in the form of eyedrops to treat herpes simplex virus conjunctivitis and intravenously or intraperitoneally as injections or infusions for the treatment of various viral infections. Eyedrops typically contained greater than about $1 \times 10^6$ units of interferon/ml (one preparation containing 60,000 units/ml was reported to confer no clinical benefit; Sundmacher et al., 1976, "J. Infect. Dis." 133: A160-A164). Intravenous doses also generally exceeded $1 \times 10^6$ units/patient, although earlier workers, constrained by the crude interferon preparations then available, necessarily used lower doses. As a result of these studies, interferons generally are not believed to be as effective in treating established viral infections as they are in preventing them. Interferon preparations are needed that demonstrate enhanced activity towards active infections and that exhibit higher protective potency in order that lower interferon doses can be used.

Tumor necrosis factor (Pennica et al., 20/27 Dec. 1984, "Nature" 312:724) and lymphotoxin (Gray et al., 20/27 Dec. 1984, "Nature" 312-721) are proteins produced by activated macrophages and lymphocytes, respectively designated "TNF" (or "TNF-$\alpha$") and "LT" ("or TNF-$\beta$") herein. Both are directly cytotoxic to tumor cells in vitro and in vivo, and synergize with interferons in this respect (Lee et al., 1984, "J.Immun."133:1083). However, neither TNF nor LT per se were known to have any direct antiviral protective activity. TNF-$\alpha$ has been suggested to be responsible for wasting and cachexia in patients with cancer of severe infections (34), and passive immunization against TNF-$\alpha$ was reported to protect mice against the lethal effect of endotoxin (30). The anti-tumor effect of TNF-$\alpha$ is known to be synergistically potentiated by interferons, and the anti-tumor effect of TNF-$\beta$ is similarly potentiated by interferon gamma. The anti-tumor effects of TNF-$\alpha$ and TNF-$\beta$ in admixture, however, are only additive, as are the antiviral effects of interferons alpha and beta.

There are a number of prior art disclosures of synergistic compositions comprising an interferon ($\alpha$, $\beta$ or $\gamma$) and tumor necrosis factor or lymphotoxin: EP-A 0 128 009 discloses a synergistic composition of interferon- with lymphotoxin; Ep-A 131 789, EP-A 170 843 and W) 86/03571 all disclose synergistic compositions of interferon ($\alpha$, $\beta$ or ) with tumor necrosis factor. All four of these documents, however, only disclose synergistic activity in relation to anticancer and antitumour therapy.

It is an object of the uses and compositions according to this invention to enhance the antiviral activity of interferons without increasing the incidence of interferon side effects.

It is a further object of the uses and compositions according to this invention to enhance the antiviral specificity of interferons.

It is an additional object to of the uses and compositions claimed herein employ interferons in the prophylaxis of individuals against viral infection, including infection by DNA viruses.

It is an object of the uses and compositions according to this invention to employ immunotherapy in the successful treatment of persons infected with retroviruses, in particular lymphocytotropic viruses such as HIV.

The uses and compositions according to this invention are suitable to treat those at risk for active retroviral infection, e.g. persons harboring latent retrovirus and to provide immunotherapeutic prophylaxis against retroviral infections.

These and other objects of the invention will be apparent from consideration of the description illustrating the features of the claims.

## Summary

The objects of this invention are accomplished by administering to a mammal previously infected or at

EP 0 235 906 B1

risk from viral infection an antivirally effective among of a TNF or an LT, of (a) an interferon and (b) a TNF and/or an LT. Notwithstanding the absence of any known antiviral protective activity by TNF or LT alone, TNF or LT synergistically enhance the antiviral activity of interferons. Typically, and quite unexpectedly, the activity of interferon is increased about from 2 to over 100 times by including TNF or LT in the interferon composition. The greatest effect is observed with gamma interferon.

Interferon compositions are provided that, at the time of this invention, would have been believed to contain inadequate interferon for therapeutic use, including either antiviral or antitumor uses. These interferon doses are less than about 500,000 International Units, generally less than 25,000 Units. They are rendered efficacious by the inclusion therein of LT or TNF in an amount in itself insufficient to demonstrate LT or TNF toxicity but sufficient for enhancing the antiviral activity of interferon.

Unexpectedly, I have discovered that administration of a therapeutically effective amount of a tumor necrosis factor along or, preferably, in combination with an interferon, confers protection on persons at risk to retroviral infection and kills retrovirally infected cells. While gamma interferon alone exerts little or no protective activity against retroviral infection nor is gamma interferon significantly cytotoxic to retrovirally infected cells, and TNF alone is only modestly active at high concentrations, the combination of these two agents is dramatically potent. This phenomenon is observed in vivo, despite the deranged condition of the immune systems of retrovirally-infected patients. This result was particularly surprising because TNF was not known to be deficient in such patients.

Brief Description of the Figures

Figs. 1 and 2 illustrate the dramatic increase in interferon-γ antiviral activity when interferon-γ is used in combination with LT or TNF.

Fig. 3 depicts a cell culture which has been protected from viral infection by varying concentrations of TNF and interferon-γ.

Figs. 4a and 4b show that the antiviral activity of interferon-α, β or γ is enhanced by TNF or LT.

Figs. 5a - 5d illustrate that inhibition of viral replication by interferon-γ is potentiated by TNF or LT.

Fig. 6 is a Northern gel showing the dramatic reduction in HIV mRNA in HIV-infected HuT78 cells after pretreatment with, TNF-α and IFN-γ as compared to control cells that were not pre treated.

Fig. 7 demonstrates the antiviral protective effect of catalase in combination with TNF-α and/or IFN-γ.

Detailed Description

The uses and compositions of this invention are useful in preventing or treating active or latent infections by DNA viruses, single stranded RNA or double stranded RNA viruses, including without limitation adenoviruses, herpes virus, papovavirus (including simian virus 40, papilloma and polyoma viruses), pox viruses such as small pox and vaccinia, arbovirus, arenovirus, coronavirus, myxovirus (New Castle disease, mumps, measles and respiratory synctial virus), rhinovirus, paramyxovirus, such as influenza viruses A, B or C, parvovirus, picornavirus, togavirus, retrovirus (including HTLV-I, II and III), reovirus and rotavirus. Other specific viruses include rubella, herpes simplex, varicells-zoster, chicken pox, cytomegalovirus, ebola virus, rabies and hepatitis B.

Retroviruses are defined as viruses containing single or double stranded RNA. These viruses replicate by harnessing the cellular metabolism of permissive hosts to reverse transcribe the RNA genetic material of the virus. The large amounts of DNA so produced are translated into HIV protein and RNA for the assembly of progeny virions. Examples of such viruses include the socalled "slow" or lentiviruses and the T-cell leukemia viruses such as HTLV-I and HTLV-II, but most preferably are the HIV (previously) known as HTLV III) strains associated with AIDS.

Interferons are well known. In nature they comprise β and γ interferon, and the about 20 different interferon-α subtypes. Their most relevant characteristic for the purposes herein is that they are capable of protecting cells in vitro and in vivo from viral infection. The interferons used in the process or composition of this invention typically are interferon-α, β or γ, with interferon-γ being preferred. Interferons produced in recombinant cell culture, from natural isolates or by stable untransformed cell lines are satisfactory for use herein, as are interferon amino acid sequence or glycosylation variants (including unglycosylated forms) so long as they exhibit anti-viral activity. Interferon-γ should be of the same animal species for which therapy is intended because interferon-γ is known to be species specific. The preferred interferon is human interferon-γ. The interferons desirably are substantially homogeneous and will have a specific activity in excess of about $1 \times 10^6$ International Units/mg.

TNF and LT include the products of recombinant or untransformed cell culture, including amino acid sequence or (in the case of LT) glycosylation variants (including unglycosylated LT). Either TNF or LT alone, or mixtures thereof, are suitable. Since TNF and LT are not species-specific in their ability to synergize with interferons, TNF or LT from one species is useful in the therapy of another. Preferably, human mature TNF or human unglycosylated mature LT are used herein.

TNF-α or TNF-β are used alone or in admixture with one another in proportions empirically determined

4

to exert the most effective clinical response. TNF is not species specific, so TNFs from other animal species, e.g. porcine or bovine, are useful herein. The preferred TNF is mature human TNF-α from recombinant microbial cell culture. The TNF ordinarily will have a cytolytic activity on susceptible L-929 murine cells of greater than about $1 \times 10^6$ units/mg, wherein a unit is defined as set forth in our earlier applications EP168214A and EP-164965A.

The compositions herein include a pharmaceutically acceptable vehicle such as those heretofore used in the therapeutic administration of interferons, TNF or LT, e.g. physiological saline or 5% dextrose, together with conventional stabilizers and/or excipients such as human serum albumin or mannitol. The compositions are provided lyophilized or as sterile aqueous solutions.

Several variables will be taken into account by the ordinary artisan in determining the proportions of interferons α, β or γ and of TNF-α or TNF-β, the net proportion of interferon to TNF, the concentration of interferon and TNF in the therapeutic compositions and the dosages to be administered on a Kg basis. Therapeutic variables also include the animal species to be treated, the administration route, and the clinical condition of the patient (including the stage and degree of retroviral and/or adventitious organism infection, if any, present at the commencement of treatment). Doses of interferon ranging about from 1 to 50 μg/m² are suitable initial dosing levels. The tolerated dose may not exceed about 25 μg/m².

Suitable doses of interferon ranging about from 50% to 0.1% of those heretofore believed to be the minimum effective doses are useful in combination with TNF or LT in conferring resistance to viral infection and for the killing of virally infected cells. These doses will range up to 500,000 International Units/70 Kg patient, but are typically less than 100,000 Units/70 Kg. The relative proportion by weight of interferon to TNF or LT generally ranges from 1000:1 to 1:1, with 100:1 being preferred. Therapeutic compositions typically are aqueous solutions containing from $1 \times 10^3$ to $1 \times 10^5$ IU of interferon/ml and from 1 ng to 5 μg of TNF or LT/ml. These are administered intranasally, intraperitoneally or intravenously, depending upon the viral infection to be interdicted or treated. Intranasal doses generally are less than 25,000 International Units (in 0.1 ml volume), while intravenous doses are less than 500,000, generally less than 100,000 Units.

The synergistic anti-viral protective effect of interferons together with TNF or LT is broadly applicable to all mammals. The cell lines shown in the following Table 1 were seeded into 24-well tissue culture plates and preincubated with TNF or LT plus interferon-γ. All were protected (as determined by cytopathic effect) to at least some degree against infection with EMC, VSV and HSV-2 at multiples of infectivity of 1, 1 and 100, respectively.

### Table 1

| A) Human Cell Lines | B) Non-Human Cells |
|---|---|
| 1. A549 (lung carcinoma) | 1. 3T3 (mouse fibroblast) |
| 2. HT-1080 (lung fibrosarcoma) | 2. C127 (mouse epithelial cells) |
| 3. Hela (cervical carcinoma) | 3. Raw-264 (mouse macrophage) |
| 4. T24 (bladder tumor) | 4. Rat-1 (rat fibroblast) |
| 5. HT29 (colon tumor) | 5. NRK (normal rat kidney) |
| 6. 7860 (renal tumor) | 6. PK15 (pig cells) |
| 7. ST486 (lymphoid cells) | 7. MDBK (bovine cells) |
| 8. R8226 (lymphoid cells) | |

Accordingly, the subject-matter of this invention is useful for conferring protection against viral infections on mammals, including man, cattle, swine, fowl and horses. Patients may or may not be known to harbor tumors or malignancies.

The uses and compositions of this invention are particularly useful in the prophylaxis of highly-communicable viral infections that may occur in dense populations of agricultural animals, the foremost being New Castle disease in chickens and shipping fever in cattle. In contradistinction to vaccines, the compositions herein are effective against all viruses, notwithstanding mutations that may occur in the viral population that could render a vaccine ineffective, and can be administered with rapid protective effect upon the first sign of an epidemic outbreak - thereby obviating the need to vaccinate an entire population of animals.

The uses and compositions also are useful in the prophylaxis of respiratory tract infections. Those at risk to exposure, e.g. those having a family member who has contracted a common cold, will administer interferon and TNF or LT for protection from infection. The most convenient route of administration for viruses transmitted in animals or man through the respiratory tract by intranasal spray or pledget. This treatment will use the known compositions and methods except that only a fraction of the interferon previously employed need be used and the therapeutic regimen includes the administration of TNF or LT. Intranasal preparations optionally include permeation enhancers such as bile acid salts or nonionic polyoxyethylene ethers. The formulations will contain the indicated doses of interferons and TNF or LT, mannitol or other stabilizers or excipients and 1% by weight enhancer in pH 7.4 phosphate buffer. Other suitable intranasal base formulations are described in U.S. patent i.e. US-A 4 476 116, EP-A 127 535, EP-A 122 036A, EP-A 111 841, and EP-A 128 831.

The TNF and interferon doses are administered together or separately. If the latter, the interferon should be administered first and the TNF thereafter within 24 hours. It is possible to administer the TNF and interferon in multiple cycles, depending upon the clinical response of the patient. This approach to the therapy will be effective in attacking latently-infected cells entering the active phase of infection, whether due to exogenously administered T cell mitogens or adventitious infections that lead to T cell activation.

Since the treatment with TNF and interferon will lyse virally-infected cells and may result in the release of infective virus, it is advantageous in the course of therapy to administer substances capable of neutralizing further viral infectivity. This can be accomplished by several methods. One can administer antibodies such as monoclonal or polyclonal anti-retroviral antibodies during the course of therapy, preferably at the same time as TNF is administered. Alternatively, immunologically competent patients can be vaccinated against the retrovirus in order to actively induce neutralizing antibody. A suitable vaccine for this purpose contains the HIV gp120 envelope polypeptide. This vaccine is disclosed to induce HIV neutralizing antibodies, which in turn can be used in the passive immunization strategy described above. Other agents that interdict the potential infectivity of released viruses also can be administered together with the TNF, for example gp120 env or fragments thereof which bind to the cell surface receptor ordinarily recognized by the retrovirus in question (in the case of HIV, the OKT4+ cell surface marker present on helper T cells) and thus competitively inhibit viral adhesion to target cell surfaces.

The synergistic antiviral and anti-tumor activity of TNF and/or an interferon is further potentiated by including in the treatment regiment and/or compositions a therapeutically effective amount of an oxygen free-radical scavenger (including oxygen protective enzymes and peroxidatively active substance). Such substances, including catalase, superoxide dismutase, peroxidase or chloroperoxidase, greatly enhance the activity of TNF and interferon. Such enzymes are respectively known to catalyze the breakdown of $H_2O_2$ to water and oxygen or to catalyze the reaction $H_2O_2 + HO-R-OH \rightarrow 2H_2O + O=R=O$, although at present the mechanism(s) by which such agents potentiate TNF and interferon are unknown. Catalase is widely available commercially and can be obtained from human tissues such as red blood cells. Peroxidase is commonly available from horseradish. The proportion of peroxidatively active substance to TNF and interferon optionally will be from 1000:1:0.1 to 100:50:25, but the dosages and proportions necessarily will be adjusted to reflect the clinical condition of the patient and administration route, among other variables known to those skilled in the art.

The TNF and interferons are administered by the same or separate routes, for example by intravenous, intranasal or intramuscular administration. Either or both components can be administered from sustained release compositions, for example as polylactide or polyhydroxylbutyrate implants or liposomes such as are described in EP 17,2007A, or by continuous infusion. At the present time it is preferred to infuse the TNF and interferon intravenously in the dosages described above.

In order to avoid side effects from the combined therapy when high doses are used, the TNF and interferon can be employed in an extracorporeal treatment regimen heretofore referred to as adoptive immunotherapy. In these methods the peripheral blood mononuclear or lymphocytic cells of a patient are separated from the blood in an extracorporeal plasmapheresis cycle, treated with interleukin-2 or interferon, and then reinfused into the patient. The patient's immune responses against malignancies were greatly stimulated by this procedure. For the treatment of retroviral infections, the same general procedure is employed except that the peripheral monocytes and/or lymphocytes are incubated in the presence of TNF or TNF and interferon. Virally-infected cells are killed by TNF or the combination of agents. The extracorporeal cells also can be incubated with a killer cell activating agent such as a lymph cell mitogen (e.g., phytohemagglutinin) and/or interleukin-2. The cells then are washed and resuspended in an infusion medium for reinfusion into the same patient from whom the cells were first obtained. The reinfusion can be followed up by administration of TNF and interferon as is otherwise provided herein. The optimal amounts of TNF or TNF and interferon, and the optimum conditions for extracorporeal treatment of the patients' lymph cells are routinely determined by assaying the patients' viral titer and assessing improvements in the patients' clinical condition.

Persons that are candidates for treatment in accordance with this invention are those at risk for exposure to retroviral infection or who exhibit signs of actual exposure to such an infection. Those at risk include members of high risk groups such as homosexuals, intravenous drug users and those who have received transfusions or other products made from blood not screened for HIV antibodies. Evidence of exposure to retrovirus includes seroconversion to antibodies against HIV, positive serum assays for HIV, symptoms associated with AIDS-related complex (ARC) or frank AIDS. AIDS patients may or may not be diagnosed for Kaposi's sarcoma or other malignancies, adventitious microbial infections such as Pneumoxystis carinii or thrush, neural impairment or cachexia (wasting).

The invention will be more fully understood in the light of the following examples. The interferons were the products of recombinent bacterial cell culture and were purified to a specific activity of $10^8$ units/mg. TNF and LT also were recombinant, and had a specific activity of $5 \times 10^7$ units/mg.

EXAMPLE 1

LT or TNF Potentiates the Antiviral Activity of Human Interferon-γ

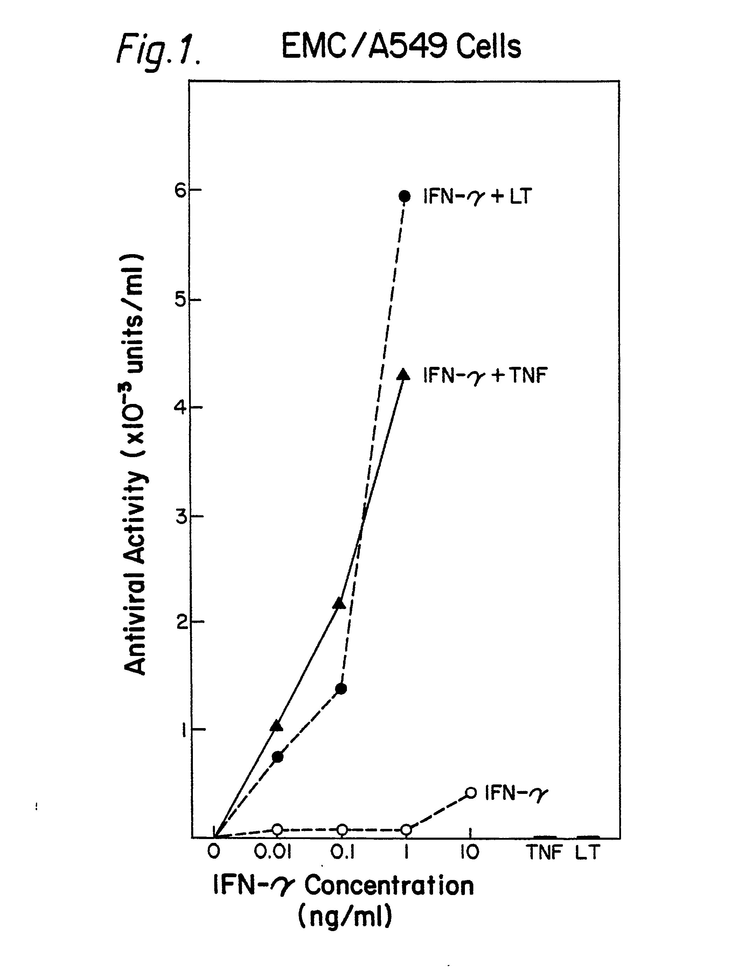

A549 cells were seeded at $2 \times 10^4$/well in 96-well flat-bottom trays (Falcon Plastics) for 24 hr prior to incubation with serially 2-fold diluted samples. The samples were dilutions of interferon-γ at the concentrations indicated in Fig. 1. The samples otherwise contained 0.1 μg/ml of TNF or LT in Dulbecco modified Eagle's (DME) medium supplemented with 5 percent heat-inactivated fetal calf serum (FCS), glutamine (2 mM), penicillin (100 U/ml), and streptomycin (100 μg/ml). Control samples contained a) no interferon, TNF or LT, b) TNF alone or c) LT alone, After 18 hr of incubation at 37°C, culture supernatants were replaced with fresh DME medium containing 2 percent fetal calf serum (but no interferon, TNF or LT) and EMC virus at a multiplicity of infection ("MOI", the ratio of infectious virus/cell) of 1. The cytopathic effect (CPE) was determined by staining the viable cells with crystal violet and the titer was quantitatively monitored using a microelisa autoreader (MR580, Dynatech) and further confirmed visually. The CPE antiviral titer is expressed as the reciprocal of the dilution found to inhibit 50 percent of the cell cytopathy and was standardized against the international reference sample of human interferon-γ (No. Gg 23-901-530).

As shown in Fig. 1, the presence of LT and TNF synergistically enhanced the anti-viral effect of interferon-γ at these interferon-γ concentrations. The anti-viral titers of interferon-γ increased greatly at concentrations above 1 ng/ml (data not shown). However, LT or TNF were very useful at conferring protection on target cells when interferon-γ was in itself present in low, largely ineffective concentrations.

EXAMPLE 2

The Antiviral Activity of Bovine Interferon-γ is enhanced by Human LT or TNF

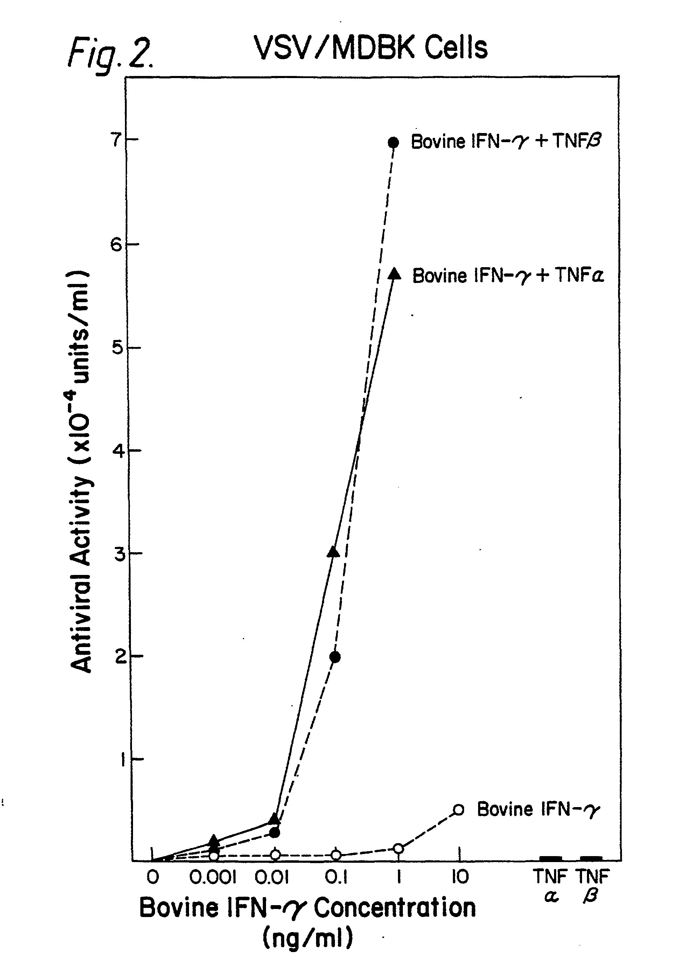

The method of Example 1 was repeated except that the TNF and LT concentrations were 1 μg/ml rather than 0.1 μg/ml, the interferon was bovine, the target cells were bovine MDBK and the virus was VSV. The results of the CPE assay are shown in Fig. 2. Once again, large increases in protection was enabled by the presence of TNF or LT. This data also shows that the species origin of the TNF or LT is unimportant for the protective effect and that it is applicable to other mammals than man.

EXAMPLE 3

Protection of A549 Cells from VSV Infection

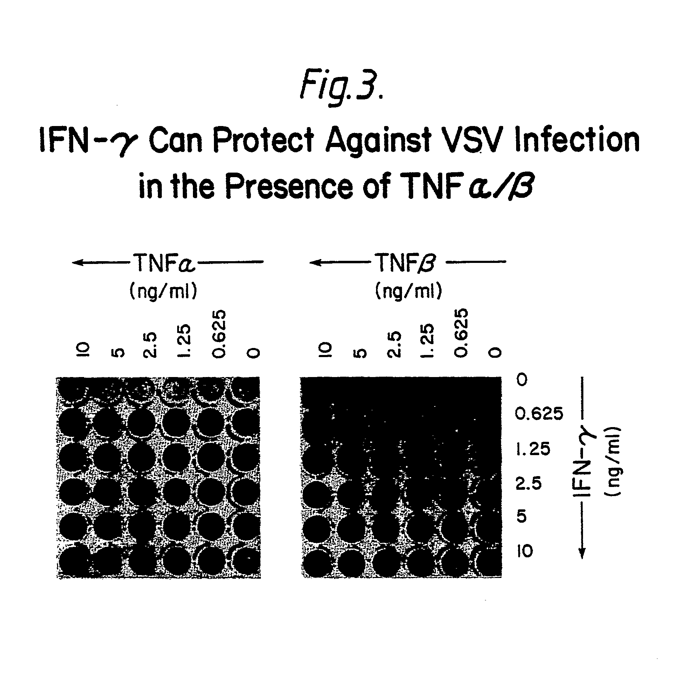

The method of Example 1 was repeated except that the test virus was VSV and serial 10-fold and 2-fold dilutions of human recombinant interferon-γ and TNF respectively, starting with 1 μg/ml of TNF, and 10 μg/ml of interferon-γ. The results are shown in Fig. 3 as a cell culture plate that has been stained with crystal violet. Concentrations of interferon-γ ranging from 0.0001 to 10 μg were ineffective in protecting the cells, as were concentrations of TNF ranging downward from 1 μg/ml to 1 ng/ml. The virally infected and uninfected controls produced the expected results as shown. However, the combination of TNF and interferon-γ protected the cells against VSV infection even at concentrations respectively ranging about from 1 μg/ml to 1 ng/ml and 10 μg/ml to 0.1 ng/ml.

EXAMPLE 4

Protective Enhancing Effect of TNF or LT on Interferons-α and β

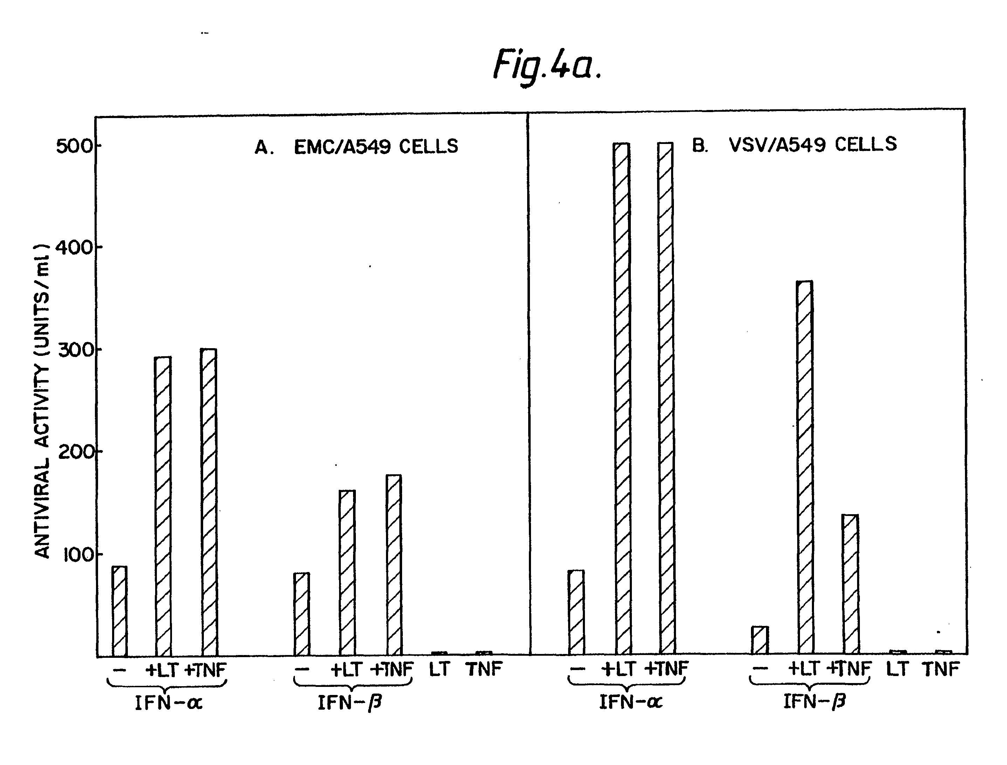

The method of Example 1 was repeated with EMC/A549 as well as VSV/A549. The interferon concentrations were 10 ng/ml of interferon-α and 10 ng/ml of interferon-β. As shown in Fig. 4a, interferon-α ·

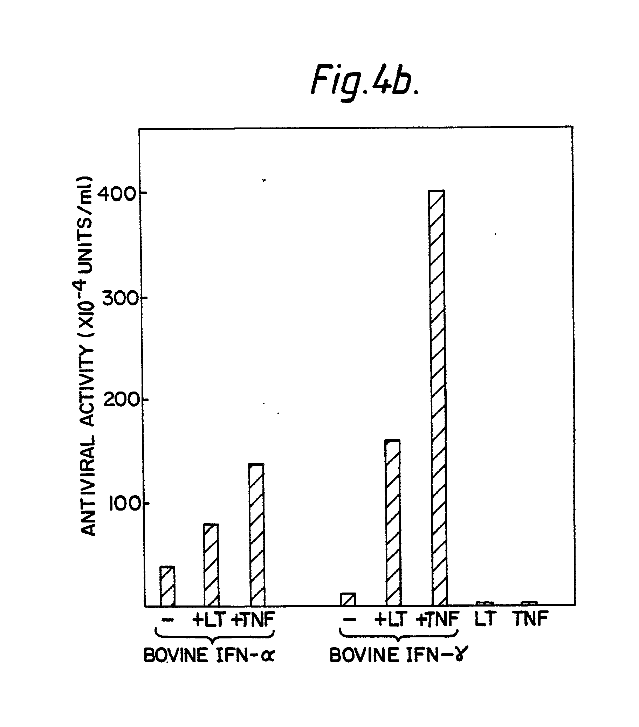

and, to a lesser degree, interferon-β demonstrated antiviral synergy with human TNF or LT. Similar results were obtained with the MDBK/VSV and bovine interferon-α and γ as shown in Fig. 4b.

## EXAMPLE 5

Synergistic Inhibition of Viral Replication

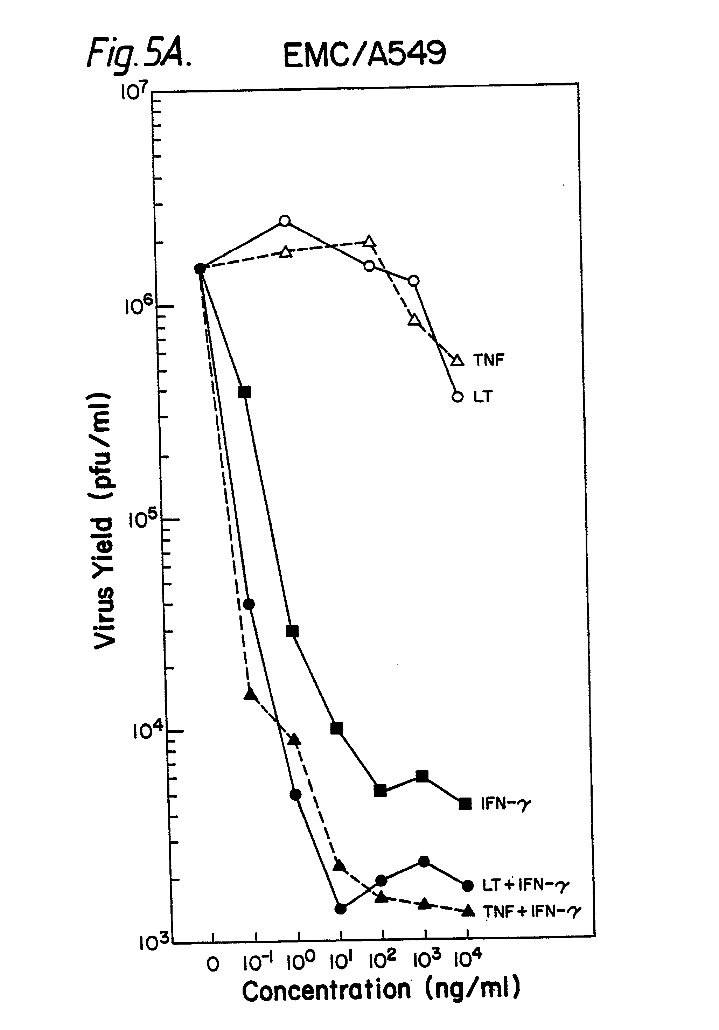

A549 cells were seeded into 24-well tissue culture plates (Costar) for 24 hr and then treated with various concentration of human LT, TNF or interferon-γ for 18 hr, and the medium removed before challenge with EMC, VSV, Adenovirus-2 (Ad-2) and Herpes Simplex-2 (HSV-2) viruses. The multiplicities of infection of EMC, VSV, Ad-2 and HSV-2 were 1, 1, 100 and 100, respectively. The concentration of TNF, LT and interferon-γ ranged from 0.1 ng/ml to 10 μg/ml. After an adsorption period of 2 hr, the supernatants were aspirated to remove the unadsorbed virus and the cells incubated at 37°C in medium containing 5 percent FCS. After 24 hr, the cultures (cells together with medium) were frozen and thawed twice to lyse the cells and then centrifuged at 400 Xg for 10 min and the virus yield determined by plaque assay as described in Rager-Zisman et al., "Proc Soc. Exp. Med." 142:1174-1179 (1973). Serial dilutions of the lysate were added to confluent A549 cells and incubated for 2 hr at 37°C. The supernatant was aspirated and the cells were then overlaid with medium containing 5 percent FCS and 0.7 percent agarose. After 24-48 hr, the plaques were visualized by fixing with formaldehyde and staining with crystal violet. The virus yields in terms of plaque forming units/ml (PFU/ml) were determined and the results shown in Figs. 5a-5d. These figures demonstrate synergistic enhancement of antiviral activity, particularly at low interferon-γ concentrations.

## EXAMPLE 6

Synergistic Killing of Virally-Infected Cells

The method of Example 1 was repeated with VSV at an MOI of 10 and without preincubation with interferon, TNF or LT. VSV was incubated at 37°C with the cells for 4 hours, after which the virus was washed from the infected cells. Then, the VSV-infected or uninfected cells were treated with 0.1 μg/ml of LT, interferon-γ, alone or in combination as shown in Table 2. After 12 and 18 hours at 37°C, the viability of the cells were determined by trypan blue or propidium iodide staining. The results are shown in Table 2 below.

## Table 2

| | Viability Count (%) | | | |
|---|---|---|---|---|
| | Uninfected | | VSV-infected | |
| Cell Treatment | 12 hr | 18 hr | 12 hr | 18 hr |
| control | ⁻100 | | 92 | 70 |
| LT | ⁻100 | | 41 | 29 |
| TNF | ⁻100 | | 38 | 31 |
| interferon-γ | ⁻100 | | 91 | 66 |
| LT + interferon-γ | ⁻100 | | 14 | 0 |
| TNF + interferon-γ | ⁻100 | | 12 | 0 |

Table 2 shows that virally infected cells were killed in synergistic fashion when combinations of interferon-γ and TNF or LT were used. Taken together with the results in previous Examples, this suggests that combinations of TNF or LT plus an interferon would act to counteract viral infections by two mechanisms: one by conferring protection on uninfected cells and the second by killing virally-infected cells.

Example 7

Prophylaxis of HIV Infection

The OKT4[+]-positive human T-cell leukemia cells lines H9, HuT78 and U937 were cultured in suspension with RPMI-1640 medium at 37°C. Cultures were centrifuged to separate cells from the medium and the cells then resuspended in Fresh RPMI-1640 at a density of $1 \times 10^6$/ml. Sufficient TNF-$\alpha$ and gamma interferon were added to the resuspended culture to produce 0.1 $\mu$g/ml TNF-$\alpha$ and 0.1 $\mu$g/ml gamma interferon. The combination of TNF-$\alpha$ and gamma interferon was not toxic to uninfected HuT78 and H9 cells. The culture then was incubated at 37°C for 24 hours, after which $1 \times 10^6$ cpm units of HIV/ml was added. The cpm units were determined by reverse transcriptase activity (31); each unit is calibrated to represent the reverse transcriptase activity of one virion. After 3 days of further incubation at 37°C the cells were screened for viral antigens by indirect immunofluoresence using a murine monoclonal antibody against the p24 (core) protein of HIV and labelled goat anti-mouse immunoglobulin (32). The results of replicate experiments are shown below as the percentage of cells which contain HIV core protein.

## Table 3

| Agent | % of Cells Infected | | | | | |
|---|---|---|---|---|---|---|
| | HuT78 | | H9 | | U937 | |
| | 1 | 2 | 1 | 2 | 1 | 2 |
| Control | 54 | 68 | 48 | 36 | 11 | 14 |
| TNF-$\alpha$ | 27 | 34 | 32 | 18 | 7 | 8 |
| gamma interferon | 63 | 51 | 42 | 41 | 12 | 11 |
| TNF-$\alpha$ and gamma interferon | 1 | 8 | 4 | 3 | 2 | 1 |

These results demonstrate that combination therapy with TNF and interferon is highly effective in preventing HIV infection of otherwise susceptible T cells.

Example 8

Treatment of HIV-Infected Cells

$1 \times 10^5$ H9 and RPMI-1788 lymphoblastoid cells/ml of RPMI-1640 medium were exposed to $1 \times 10^3$ cpm of HIV/ml in the presence of 1 $\mu$g/ml polybrene in order to enhance the percentage of cells infected by HIV. These cells were cultured for about 30 days, during which the culture medium was replaced every 3-5 days and cells subcultured approximately once a week. Sufficient TNF-$\alpha$ and gamma interferon were substantially simultaneously added to cultures containing $1 \times 10^6$ cells/ml in order to establish concentrations as shown in Table 4 below. Combinations of TNF-$\alpha$ and gamma interferon at these concentrations were not cytotoxic towards uninfected H9 and RPMI-1788 cells. The cultures were incubated at 37°C for 3 days and then assayed for the percentage of viable cells by trypan blue staining. The results of replicate experiments are shown in Table 4 (ND = not done).

9

## Table 4

| Agent | Concentration | H9 | | 1788 | |
|---|---|---|---|---|---|
| | | 1 | 2 | 1 | 2 |
| Control | -- | 89 | 76 | 95 | 86 |
| TNF-α | 1.0 ng/ml | ND | ND | 90 | 81 |
| | 0.1 μg/ml | 73 | 70 | 81 | 76 |
| Gamma interferon | 1.0 ng/ml | ND | ND | 83 | 84 |
| | 0.1 μg/ml | 85 | 74 | 80 | 82 |
| TNF-α and Gamma interferon | 1.0 ng/ml | ND | ND | 68 | 72 |
| | 0.1 μg/ml | 50 | 42 | 34 | 53 |

These results demonstrate concentration-dependent synergistic killing of virally-infected cells by combinations of TNF and interferon and, to a lesser degree, TNF-α alone. This, combined with the protective effect of TNF or the combinations for uninfected cells, demonstrates the value of TNF or combinations therapy in the treatment of retroviral infections.

Example 9

Reduction of HIV Replication by TNF and Combination Therapy

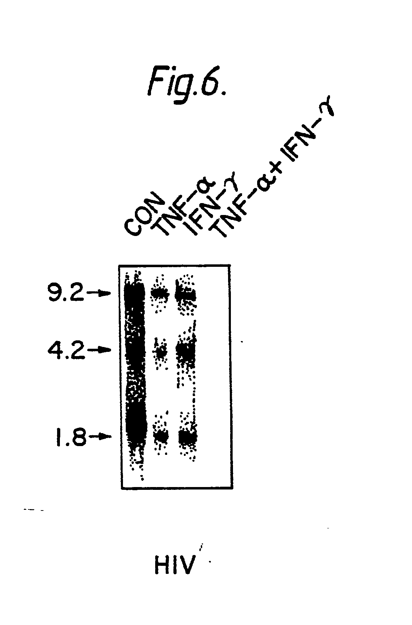

HuT78 cells were treated with 0.1 μg/ml each of TNF-α and gamma interferon and then infected with HIV as described in Example 7. After 2 days of incubation at 37°C total RNA was extracted from the HIV-infected cells as follows. The cells were washed with PBS and resuspended in 0.35 ml TSM buffer (10 mM Tris pH 7.5, 0.15 M NaCl, 2mM MgCl₂) containing 0.5% NP-40 and 17 μl VRC (BRL) at about 0°C. After 3-5 min. nuclei were centrifuged free of the lysed cells. The supernatant containing mRNA was added to 0.35 ml of TSE buffer (10 mM Tris pH 7.5, 0.15 M NaCl, 5 mM EDTA) containing 1% SDS and extracted 3x with 0.7 ml of phenol:chloroform:iso-amylalcohol (24:24:1). The phenol was equilibrated with water and 0.1% 8-OH-quinoline. RNA was precipitated from the aqueous layer with ETOH and sodium acetate. Poly(A) RNA was prepared by oligo(dT) cellulose electrophoresis (36). Northern hybridization using 1 μg RNA/lane was performed as described (37). The results, shown in Fig. 6, demonstrate that TNF-α pretreatment was able to suppress the appearance of HIV RNA in host cells, but that far less RNA appeared when TNF and interferon were used together. These results are consistent with the infectivity data shown in Table 3. No change in actin mRNA (a structural protein) was observed with or without TNF and interferon treatment, demonstrating that the effect of TNF-α and interferon was targeted to the virus rather than cell growth in general.

Example 9

Protocol for AIDS or ARC Patient Therapy

Males seropositive for HIV antibody and having blood which is in vitro cell culture positive for demonstrable HIV titer are enrolled. These patients generally present with symptomology consistent with AIDs or ARC, and were seroconverted for HIV antibody. The treatment group is divided into cohorts based on the following treatment variables:

1. Simultaneous or sequential TNF and interferon treatment.

2. Intravenous or intramuscular injection and, in the case of intravenous injection, by bolus or continuously by infusion (1, 2, 5 and 10 days).

3. Dosage regimen over hours, starting at 1 $\mu$g/m$^2$ of TNF, and 1 $\mu$g/m$^2$ of interferon, and increasing to 5, 10, 25, 50 ... $\mu$g/m$^2$ of each agent as tolerated.

4. Ratios of TNF to interferon: 1:100 to 100:1.

5. Repeat cycles of treatments: 1 to 5, with intermediate treatment suspensions of 1, 2, 5 or 10 days.

6. Selection and proportions of interferon types: $\alpha$ and $\gamma$, 1:10 - 10:1; $\beta$ and $\gamma$, 1:10 - 10:1; $\gamma$, $\alpha$ or $\beta$ alone, 1:1:1 to 10:1:1 to 1:5:5.

A suitable starting regiment will comprise treating ARC patients with a combination of TNF-$\alpha$, gamma interferon and alpha interferon. The combination is administered by continuous infusion using an intravenous infusion pump calibrated to deliver a dosage of 1-10 $\mu$g/m$^2$/24 hours TNF-$\alpha$, 1-10 $\mu$g/m$^2$/24 hours for gamma interferon and 1-10 $\mu$g/m$^2$/24 hours for alpha interferon. Patients may be escalated to higher doses as tolerated. This infusion is continued for one week. Patients are rested for another week and the infusion repeated. Side effects such as fever or chills are treated by conventional means or by reducing the dosage. When anti-HIV antibody or pg120 env are employed as antiviral agents in combination with the above, the antibody or env polypeptide are present in a dosage calculated to be capable of sequestering virions released by the combined therapy. This will depend on the affinity of antibody for virion and its neutralizing titer as well as the viral titer of the patient. Determination of suitable dosages will be within the skill of the routineer.

The clinical condition and viral infectivity titers of the patients are monitored during and after the treatment protocol. Consistent with the in vitro studies in Examples 7-9, the immune competence and viral titer of a statistically significant proportion of the patients improved as a result of the treatment.

Example 10

Catalase Potentiates the Antiviral Activity of Interferon-$\gamma$ and/or TNF-$\alpha$

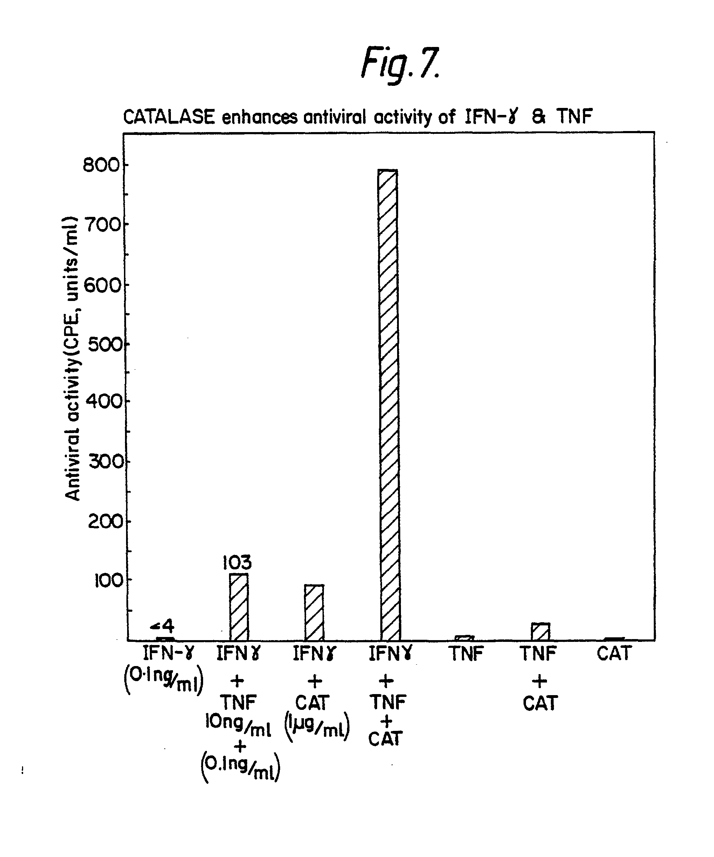

A549 cells were seeded at 2x10$^4$/well in 96-well flat-bottom trays (Falcon Plastics) for 24 hr prior to incubation with test samples. The test samples were as shown in Fig. 7 ("CAT" is catalase). The samples otherwise contained Dulbecco modified Eagle's (DME) medium supplemented with 5 percent heat-inactivated fetal calf serum (FCS), glutamine (2 mM), penicillin (100 U/ml), and streptomycin (100 $\mu$g/ml). After 18 hr of incubation at 37°C, culture supernatants were replaced with fresh DMA medium free of interferon, TNF or catalase but containing 2 percent fetal calf serum and EMC virus at a multiplicity of infection ("MOI", the ratio of infectious virus/cell) of 1. The cytopathic effect (CPE) was determined by staining the viable cells with crystal violet and the titer was quantitatively monitored using a microelisa autoreader (MR580, Dynatech) and further confirmed visually. The CPE antiviral titer is expressed as the reciprocal of the dilution found to inhibit 50 percent of the cell cytopathy and was standardized against the international reference sample of human interferon-$\gamma$ (No. Gg 23-901-530).

As shown in Fig. 7, catalase dramatically and synergistically enhanced the anti-viral effect of interferon-$\gamma$ and TNF-$\alpha$. This effect is not unique to EMC virus. It has been observed with a variety of permissive cells and viruses.

BIBLIOGRAPHY

1. Gottlieb, M. et al., "New Engl. J. Med." 305:1425-1431(1981).
2. Masur, H. et al "New Engl. J. Med." 305:1431-1438(1981).
3. Siegal, F. et al., "New Engl. J. Med." 305:1439-1444(1981).
4. Seligman, M. et al., "New Engl. J. Med." 311:1286-1292(1984).
5. Lane, H., Masur, H., Edgar, G., Whalen, G. and Fauci, A. "New Engl. J. Med." 309:453-458(1983).
6. Ziegler, J. et al., "New Engl. J. Med." 311:565-570(I1984).
7. Barre-Sinoussi, F. et al. "Science" 220:868(1983).
8. Montagnier, L. et al. "Human T-cell Leukemia Viruses" 363-379 (Cold Spring Harbor Laboratory, New York, 1984).
9. Vilmer, E. et al. "Lancet" I: 753-757(1984).
10. Popovic, M., Sarngadharan, M., Read, E. and Gallo, R., "Science" 224:497-500(1984).
11. Gallo, R. et al. "Science" 224:500-503(1984).
12. Feorino, P. et al. "Science" 225:69-72(1984).
13. Levy, J. et al. "Science" 225:840-842(1984).
14. Klatzmann, D. et al. "Science" 225:59-63(1984).
15. Schupbach, J. et al. "Science" 224:503-505(1984).
16. Sarngadharan, M., Popovic, M., Bruch, L., Schupbach, J. and Gallo, R. "Science" 224:505-508(1984).

17. Safai, B. et al. "Lancet" I:1438-1440(1984).
18. Brun-Vezinet, F. et al. "Lancet" I:1253-1256(1984).
19. Brun-Vezinet, F. et al. "Science" 226:453-456(1984).
20. Goedert, J. et al. "Lancet" II:711-715(1984).
21. Lawrence, J. et al. "New Engl. J. Med." 311:1269-1273(1984).
22. Murray, H. et al., "New Engl. J. Med." 310:883-889(1984).
23. Fauci, A. et al. "Ann. Int. Med" 100:92-106(1984).
24. Nathan, C. et al. "J. Exp. Med." 158:670-689(1983).
25. Aderka, et al. "Cell. Immun." 92:218-225(1985).
26. Pennington, J. et al. "J. Infect. Dis." 153:609-612(March, 1986).
27. Lane, H. et al. "Clinical Research" 32:351A(1984).
28. Klatzmann, D. et al. "Nature" 319:10-11(January, 1986).
29. Yamamoto, J. et al"J. Interferon Res." 6:143-152(June, 1986).
30. Beutler, B. et al. "Science" 229:869 (30 August 1985).
31. Hoffman, A. et al. "Virology" 147:326-335(1985).
32. Kaminsky, L. et al"Proc. Nat. Acad. Sci. USA" 82:5535-5539(1985).
33. Meussing, M. et al. "Nature" 313:450-458 (1985).
34. Anonymous "Science" 229:811 (30 August 1985).
35. Ruddle, N. et al. "Immunology Today" 1:8-9(1986).
36. Aviv, H. et al. "Proc.Nat.Acad.Sci. USA" 69:1408-1412 (1972).
37. Thomas, P. "Proc.Nat.Acad.Sci USA" 77:5201-5205(1980).

**Claims for the following Contracting States: BE, CH, DE, FR, GB, IT, LI, LU, NL, SE**

1. The use of (a) an interferon and (b) a TNF and/or an LT in the manufacture of an anti-viral or prophylactic medicament for administration to a mammal previously infected or at risk from viral infection, and wherein (a) and (b) are administrable simultaneously or sequentially.

2. The use of claim 1 wherein the viral infection is a respiratory tract infection.

3. The use of claim 1 or claim 2 wherein the interferon and TNF or LT are administered simultaneously.

4. The use of any one of claims 1, 2 and 3 wherein the interferon and TNF or LT are administered intranasally.

5. The use of any one of claims 1 to 4 wherein the TNF or LT are administered in an effective dose of less than 5 μg/kg.

6. The use of a tumor necrosis factor or a tumor necrosis factor and an interferon in the manufacture of a medicament for the treatment or prophylaxis of viral infections in an animal having such an infection ar at risk from such an infection, with the proviso that when tumor necrosis factor is the sole active ingredient the medicament is for the treatment or prophylaxis of HIV viral injection.

7. The use of claim 6 wherein the tumor necrosis factor is tumor necrosis factor-α.

8. The use of claim 6 or claim 7 wherein the interferon is interferon α, β or γ.

9. The use of claim 8 wherein the interferon is interferon-γ.

10. The use of claim 6 wherein interferon is a mixture of gamma interferon plus alpha or beta interferon.

11. The use of any one of claims 6 to 9 in connection with infection by HIV.

12. The use of any one of claims 6 to 10 wherein the medicament is for administration ex vivo in the course of adoptive immunotherapy.

13. The use of any one of claims 6 to 11 wherein interferon is to be administered prior to tumor necrosis factor.

14. The use of any one of claims 6 to 13 wherein the dosages of tumor necrosis factor and interferon are each about from 1 to 25 μg/m²/24 hours by continuous intravenous infusion.

15. The use of any one of claims 6 to 14, wherein the medicament further comprises antibody capable of neutralising viral infectivity.

16. The use of claim 11 wherein the administration of tumor necrosis factor and interferon is to be repeated through about from 1 to 5 cycles.

17. The use of claim 11, wherein the medicament comprises means for immunizing the animal against HIV prior to administration of tumor necrosis factor or tumor necrosis factor and an interferon.

18. The use of any one of claims 6 to 17 wherein the medicament further comprises a physiological acceptable oxygen free-radical scavenger substance.

19. The use of claim 18 wherein the scavenger substance is catalase.

20. The use of claim 19 wherein the scavenger substance is human erythrocyte catalase.

21. A composition comprising a tumor necrosis factor, an interferon and an oxygen free-radical scavenger substance.

22. The composition of claim 21 wherein the scavenger substance is a peroxidatively active enzyme.

23. The composition of claim 22 wherein the scavenger substance is catalase.

24. The composition of claim 23 wherein the enzyme is human erythrocyte catalase.

25. The composition of any one of claims 21 to 24 further, comprising a substance capable of preventing (a) replication of a retrovirus or (b) retroviral binding to a cell surface receptor.

26. A composition comprising a tumor necrosis factor, an interferon and a substance capable of preventing the (a) replication of a retrovirus or (b) retroviral binding to a cell surface receptor.

27. The composition of claim 25 or claim 26 wherein the substance capable of preventing retroviral binding to a cell surface receptor is an antibody directed against an envelope polypeptide of the retrovirus.

**Claims for the following Contracting States: AT, GR, ES**

1. The use of (a) an interferon and (b) a TNF and/or an LT in the manufacture of an anti-viral or prophylactic medicament for administration to a mammal previously infected or at risk from viral infection, and wherein (a) and (b) are administrable simultaneously or sequentially.

2. The use of claim 1 wherein the viral infection is a respiratory tract infection.

3. The use of claim 1 or claim 2 wherein the interferon and TNF or LT are administered simultaneously.

4. The use of any one of claims 1, 2 and 3 wherein the interferon and TNF or LT are administered intranasally.

5. The use of any one of claims 1 to 4 wherein the TNF or LT are administered in an effective dose of less than 5 µg/kg.

6. The use of a tumor necrosis factor or a tumor necrosis factor and an interferon in the manufacture of a medicament for the treatment or prophylaxis of viral infections in an animal having such an infection or at risk from such an infection, with the proviso that when tumor necrosis factor is the sole active ingredient the medicament is for the treatment or prophylaxis of HIV viral infection.

7. The use of claim 6 wherein the tumor necrosis fatcor is tumor necrosis factor-$\alpha$.

8. The use of claim 6 or claim 7 wherein the interferon is interferon $\alpha$, $\beta$ or $\gamma$.

9. The use of claim 8 wherein the interferon is interferon-$\gamma$.

10. The use of claim 6 wherein interferon is a mixture of gamma interferon plus alpha or beta interferon.

11. The use of any one of claims 6 to 9 in connection with infection by HIV.

12. The use of any one of claims 6 to 10 wherein the medicament is for administration ex vivo in the course of adoptive immunotherapy.

13. The use of any one of claims 6 to 11 wherein interferon is to be administered prior to tumor necrosis factor.

14. The use of any one of claims 6 to 13 wherein the dosages of tumor necrosis factor and interferon are each about from 1 to 25 µg/m²/24 hours by continuous intravenous infusion.

15. The use of any one of claims 6 to 14, wherein the medicament further comprises antibody capable of neutralising viral infectivity.

16. The use of claim 11 wherein the administration of tumor necrosis factor and interferon is to be repeated through about from 1 to 5 cycles.

17. The use of claim 11, wherein the medicament comprises means for immunizing the animal against HIV prior to administration of tumor necrosis factor or tumor necrosis factor and an interferon.

18. The use of any one of claims 6 to 17 wherein the medicament further comprises a physiological acceptable oxygen free-radical scavenger substance.

19. The use of claim 18 wherein the scavenger substance is catalase.

20. The use of claim 19 wherein the scavenger substance is human erythrocyte catalase.

21. Use of a tumor necrosis factor, an interferon and an oxygen free-radical scavenger substance in the manufacture of a medicament.

22. The use of claim 21 wherein the scavenger substance is a peroxidatively active enzyme.

23. The use of claim 22 wherein the scavenger substance is catalase.

24. The use of claim 23 wherein the enzyme is human erythrocyte catalase.

25. The use of any one of claims 21 to 24 wherein the medicament further comprises a substance capable of preventing (a) replication of a retrovirus or (b) retroviral binding to a cell surface receptor.

26. Use of a tumor necrosis factor, an interferon and a substance capable of preventing the (a) replication of a retrovirus or (b) retroviral binding to a cell surface receptor in the manufacture of a medicament.

27. The use of claim 25 or claim 26 wherein the substance capable of preventing retroviral binding to a cell surface receptor is an antibody directed against an envelope polypeptide of the retrovirus.

**Patentansprüche für die Vertragsstaaten: BE, CH, DE, FR, GB, IT, LI, LU, NL, SE**

1. Verwendung (a) eines Interferons und (b) eines TNF und/oder LT für die Herstellung eines Antivirus- oder prophylaktischen Arzneimittels für die Verabreichung an ein Säugetier, das vorher mit Viren infiziert wurde oder die Gefahr einer solchen Infektion läuft, und worin (a) und (b) gleichzeitig oder aufeinanderfolgend verabreichbar sind.

2. Verwendung nach Anspruch 1, worin die Virusinfektion eine Infektion der Atmungswege ist.

3. Verwendung nach Anspruch 1 oder 2, worin das Interferon und TNF oder LT gleichzeitig verabreicht wird.

4. Verwendung nach einem der Ansprüche 1, 2 und 3, worin das Interferon und TNF oder LT intranasal verabreicht wird.

5. Verwendung nach einem einer Ansprüche 1 bis 4, worin das TNF oder LT in einer wirksamen Dosis von weniger als 5 µg/kg verabreicht wird.

6. Verwendung eines Tumornekrosefaktors oder eines Tumornekrosefaktors und eines Interferons bei der Herstellung eines Arzneimittels für die Behandlung oder Prophylaxe von Virusinfektion bei einem Tier, das an einer solchen Infektion leidet oder die Gefahr einer solchen Infektion läuft, mit der Maßgabe, daß wenn der Tumornekrosefaktor der einzige Wirkstoff ist, das Arzneimittel für die Behandlung oder Prophylaxe von HIV-Virusinfektionen bestimmt ist.

7. Verwendung nach Anspruch 6, worin der Tumornekrosefaktor Tumornekrosefaktor-α ist.

8. Verwendung nach Anspruch 6 oder 7, worin das Interferon Interferon-α, β, oder γ ist.

9. Verwendung nach Anspruch 8, worin das Interferon Interferon-γ ist.

10. Verwendung nach Anspruch 6, worin das Interferon eine Mischung aus Gamma-Interferon plus Alpha- oder Beta-Interferon ist.

11. Verwendung nach einem der Ansprüche 6 bis 9 in Verbindung mit der Infektion durch HIV.

12. Verwendung nach einem der Ansprüche 6 bis 10, worin das Arzneimittel für die Verabreichung ex vivo im Verlauf der adoptiven Immuntherapie bestimmt ist.

13. Verwendung nach einem der Ansprüche 6 bis 11, worin das Interferon vor dem Tumornekrosefaktor verabreicht wird.

14. Verwendung nach einem der Ansprüche 6 bis 13, worin die Dosierungsmengen an Tumornekrosefaktor und Interferon jeweils von etwa 1 bis 25 µg/m²/24 Stunden durch kontinuierliche intravenöse Infusion betragen.

15. Verwendung nach einem der Ansprüche 6 bis 14, worin das Medikament weiters Antikörper enthält, der dazu geeignet ist, Virusinfektivität zu neutralisieren.

16. Verwendung nach Anspruch 11, worin die Verabreichung von Tumornekrosefaktor und Interferon durch etwa 1 bis 5 Zyklen wiederholt wird.

17. Verwendung nach Anspruch 11, worin das Arzneimittel Mittel zur Immunisierung des Tieres gegen HIV vor der Verabreichung des Tumornekrosefaktors oder des Tumornekrosefaktors und eines Interferons enthält.

18. Verwendung nach einem der Ansprüche 6 bis 17, worin das Arzneimittel weiters eine physiologisch verträgliche Fängersubstanz für freie Sauerstoffradikale enthält.

19. Verwendung nach Anspruch 18, worin die Fängersubstanz Catalase ist.

20. Verwendung nach Anspruch 19, worin die Fängersubstanz Human-Erythrozyt-Catalase ist.

21. Zusammensetzung, die einen Tumornekrosefaktor, ein Interferon und eine Fängersubstanz für freie Sauerstoffradikale enthält.

22. Zusammensetzung nach Anspruch 21, worin die Fängersubstanz ein peroxidativ aktives Enzym ist.

23. Zusammensetzung nach Anspruch 22, worin die Fängersubstanz Catalase ist.

24. Zusammensetzung nach Anspruch 23, worin das Enzym Human-Erythrozyt-Catalase ist.

25. Zusammensetzung nach einem der Ansprüche 21 bis 24, weiters eine Substanz enthaltend, die dazu geeignet ist, (a) die Replikation eines Retrovirus oder (b) die Bindung eines Retrovirus an einen Zellenoberflächenrezeptor zu verhindern.

26. Zusammensetzung enthaltend einen Tumornekrosefaktor, ein Interferon und eine Substanz, die dazu geeignet ist, (a) die Replikation eines Retrovirus oder (b) die Bindung eines Retrovirus an einen Zellenoberflächenrezeptor zu verhindern.

27. Zusammensetzung nach Anspruch 25 oder 26, worin die Substanz, die dazu geeignet ist, die Bindung des Retrovirus an einen Zellenoberflächenrezeptor zu verhindern, ein Antikörper ist, der gegen ein Hüllpolypeptid des Retrovirus gerichtet ist.

**Patentansprüche für die Vertragsstaaten: AT, GR, ES**

1. Verwendung (a) eines Interferons und (b) eines TNF und/oder LT für die Herstellung eines Antivirus- oder prophylaktischen Arzneimittels für die Verabreichung an ein Säugetier, das vorher mit Viren infiziert wurde oder die Gefahr einer solchen Infektion läuft, und worin (a) und (b) gleichzeitig oder aufeinanderfolgend verabreichbar sind.

2. Verwendung nach Anspruch 1, worin die Virusinfektion eine Infektion der Atmungswege ist.

3. Verwendung nach Anspruch 1 oder 2, worin das Interferon und TNF oder LT gleichzeitig verabreicht wird.

4. Verwendung nach einem der Ansprüche 1, 2 und 3, worin das Interferon und TNF oder LT intranasal verabreicht wird.

5. Verwendung nach einem einer Ansprüche 1 bis 4, worin das TNF oder LT in einer wirksamen Dosis von weniger als 5 µg/kg verabreicht wird.

6. Verwendung eines Tumornekrosefaktors oder eines Tumornekrosefaktors und eines Interferons bei der Herstellung eines Arzneimittels für die Behandlung oder Prophylaxe von Virusinfektion bei einem Tier, das an einer solchen Infektion leidet oder die Gefahr einer solchen Infektion läuft, mit der Maßgabe, daß wenn der Tumornekrosefaktor der einzige Wirkstoff ist, das Arzneimittel für die Behandlung oder Prophylaxe von HIV-Virusinfektionen bestimmt ist.

7. Verwendung nach Anspruch 6, worin der Tumornekrosefaktor Tumornekrosefaktor-α ist.

8. Verwendung nach Anspruch 6 oder 7, worin das Interferon Interferon-α, β oder γ ist.

9. Verwendung nach Anspruch 8, worin das Interferon Interferon-γ ist.

10. Verwendung nach Anspruch 6, worin das Interferon eine Mischung aus Gamma-Interferon plus Alpha- oder Beta-Interferon ist.

11. Verwendung nach einem der Ansprüche 6 bis 9 in Verbindung mit der Infektion durch HIV.

12. Verwendung nach einen der Ansprüche 6 bis 10, worin das Arzneimittel für die Verabreichung ex vivo im Verlauf der adoptiven Immuntherapie bestimmt ist.

13. Verwendung nach einem der Ansprüche 6 bis 11, worin das Interferon vor dem Tumornekrosefaktor verabreicht wird.

14. Verwendung nach einem der Ansprüche 6 bis 13, worin die Dosierungsmengen an Tumornekrosefaktor und Interferon jeweils von etwa 1 bis 25 $\mu g/m^2/24$ Stunden durch kontinuierliche intravenöse Infusion betragen.

15. Verwendung nach einem der Ansprüche 6 bis 14, worin das Medikament weiters Antikörper enthält, der dazu geeignet ist, Virusinfektivität zu neutralisieren.

16. Verwendung nach Anspruch 11, worin die Verabreichung von Tumornekrosefaktor und Inrerferon durch etwa 1 bis 5 Zyklen wiederholt wird.

17. Verwendung nach Anspruch 11, worin das Arzneimittel Mittel zur Immunisierung des Tieres gegen HIV vor der Verabreichung des Tumornekrosefaktors oder des Tumornekrosefaktors und eines Interferons enthält.

18. Verwendung nach einen der Ansprüche 6 bis 17, worin das Arzneimittel weiters eine physiologisch verträgliche Fängersubstanz für freie Sauerstoffradikale enthält.

19. Verwendung nach Anspruch 18, worin die Fängersubstanz Catalase ist.

20. Verwendung nach Anspruch 19, worin die Fängersubstanz Human-Erythrozyt-Catalase ist,

21. Verwendung eines Tumornekrosefaktors, eines Interferons und einer Fängersubstanz für freie Sauerstoffradikale für die Herstellung eines Arzneimittels.

22. Verwendung nach Anspruch 21, worin die Fängersubstanz ein peroxidativ aktives Enzym ist.

23. Verwendung nach Anspruch 22, worin die Fängersubstanz Catalase ist.

24. Verwendung nach Anspruch 23, worin das Enzym Human-Erythrozyt-Catalase ist.

25. Verwendung nach einem der Ansprüche 21 bis 24, worin das Arzneimittel weiters eine Substanz enthält, die dazu geeignet ist, (a) die Replikation eines Retrovirus oder (b) die Bindung eines Retrovirus an einen Zellenoberflächenrezeptor zu verhindern.

26. Verwendung eines Tumornekrosefaktors, eines Interferons und einer Substanz, die dazu geeignet ist, (a) die Replikation eines Retrovirus oder (b) die Bindung eines Retrovirus an einen Zellenoberflächenrezeptor zu verhindern, zur Herstellung eines Arzneimittels.

27. Verwendung nach Anspruch 25 oder 26, worin die Substanz, die dazu geeignet ist, die Bindung des Retrovirus an einen Zellenoberflächenrezeptor zu verhindern ein Antikörper ist, der gegen ein Hüllpolypeptid des Retrovirus gerichtet ist.

## Revendications pour les Etats Contractants: BE, CH, DE, FR, GB, IT, LI, LU, NL, SE

1. Utilisation (a) d'un interféron et (b) d'un TNF et/ou d'un LT dans la fabrication d'un médicament prophylactique ou antiviral pour une administration à un mammifère préalablement infecté ou présentant un risque d'infection virale, et où (a) et (b) sont administrés simultanément ou en séquence.

2. Utilisation selon la revendication 1, où l'infection virale est une infection du conduit respiratoire.

3. Utilisation selon la revendication 1 ou 2, où l'interféron et le TNF ou LT sont administrés simultanément.

4. Utilisation selon l'une des revendications 1, 2 et 3, où l'interféron et le TNF ou LT sont administrés de manière intranasale.

5. Utilisation selon l'une des revendications 1 à 4, où le TNF ou LT sont administrés dans une dose efficace inférieure à 5 $\mu g/kg$.

6. Utilisation d'un facteur de nécrose des tumeurs ou d'un facteur de nécrose des tumeurs et d'un interféron dans la fabrication d'un médicament pour le traitement ou la prophylaxie d'infections virales chez un animal ayant une telle infection ou présentant le risque d'avoir une telle infection, à la condition que lorsque le facteur de nécrose des tumeurs est le seul ingrédient actif, le médicament soit pour le traitement ou la prophylaxie de l'infection virale par HIV.

7. Utilisation selon la revendication 6, où le facteur de nécrose des tumeurs est le facteur-α de nécrose des tumeurs.

8. Utilisation selon la revendication 6 ou 7, où l'interféron est l'interféron α, β ou γ.

9. Utilisation selon la revendication 8, où l'interféron est l'interféron-γ.

10. Utilisation selon la revendication 6, où l'interféron est un mélange d'interféron gamma plus un interféron alpha ou bêta.

11. Utilisation selon l'une des revendications 6 à 9, en ce qui concerne l'infection par HIV.

12. Utilisation selon l'une des revendications 6 à 10, où le médicament est pour l'administration <u>ex vivo</u> au cours de l'immunothérapie adoptive.

13. Utilisation selon l'une des revendications 6 à 11, où l'interféron doit être administré avant le facteur de nécrose des tumeurs.

14. Utilisation selon l'une des revendications 6 à 13, où les dosages du facteur de nécrose des tumeurs et de l'interféron sont chacun d'environ 1 à 25 $\mu g/m^2/$ 24 heures par perfusion intraveineuse en continu.

15. Utilisation selon l'une des revendications 6 à 14, où le médicament comprend de plus un anticorps capable de neutraliser l'infection virale.

16. Utilisation selon la revendication 11, où l'administration du facteur de nécrose des tumeurs et de l'interféron doit être répétée au cours d'environ 1 à 5 cycles.

17. Utilisation selon la revendication 11, où le médicament comprend des moyens pour immuniser l'animal contre le HIV avant l'administration du facteur de nécrose des tumeurs ou du facteur de nécrose des tumeurs et d'un interféron.

18. Utilisation selon l'une des revendications 6 à 17, où le médicament comprend de plus une substance inhibitrice à radicaux libres de l'oxygène physiologiquement acceptable.

19. Utilisation selon la revendication 18, où la substance inhibitrice est la catalase.

20. Utilisation selon la revendication 19, où la substance inhibitrice est la catalase d'érythrocyte humain.

21. Composition comprenant un facteur de nécrose des tumeurs, un interféron et une substance inhibitrice à radicaux libres de l'oxygène.

22. Composition selon la revendication 21, où la substance inhibitrice est une enzyme active de manière peroxydante.

23. Composition selon la revendication 22, où la substance inhibitrice est la catalase.

24. Composition selon la revendication 23, où l'enzyme est une catalase d'érythrocyte humain.

25. Composition selon l'une des revendications 21 à 24, comprenant de plus une substance capable de prévenir (a) la réplication d'un rétrovirus ou (b) la liaison rétrovirale à un récepteur de surface cellulaire.

26. Composition comprenant un facteur de nécrose des tumeurs, un interféron et une substance capable de prévenir (a) la réplication d'un rétrovirus ou (b) la liaison rétrovirale à un récepteur de surface cellulaire.

27. Composition selon la revendication 25 ou 26, où la substance capable de prévenir la liaison rétrovirale à un récepteur de surface cellulaire est un anticorps dirigé contre un polypeptide d'enveloppe du rétrovirus.

## Revendications pour les Etats Contractants: AT, GR, ES

1. Utilisation (a) d'un interféron et (b) d'un TNF et/ou d'un LT dans la fabrication d'un médicament prophylacti que ou antiviral pour une administration à un mammifère préalablement infecté ou présentant le risque d'une infection virale, et où (a) et (b) sont administrés simultanément ou en séquence.

2. Utilisation selon la revendication 1, où l'infection virale est une infection du conduit respiratoire.

3. Utilisation selon la revendication 1 ou 2, où l'interféron et TNF ou LT sont administrés simultanément.

4. Utilisation selon l'une des revendications 1, 2 et 3, où l'interféron et le TNF ou LT sont administrés de manière intranasale.

5. Utilisation selon l'une des revendications 1 à 4, où le TNF ou LT sont administrés dans une dose efficace inférieure à 5 $\mu g/kg$.

6. Utilisation d'un facteur de nécrose des tumeurs ou d'un facteur de nécrose des tumeurs et d'un interféron dans la fabrication d'un médicament pour le traitement ou la prophylaxie des infections virales chez un animal ayant une telle infection ou présentant le risque d'une telle infection, à la condition que lorsque le facteur de nécrose des tumeurs est le seul ingrédient actif, le médicament soit pour le traitement ou la prophylaxie d'une infection virale par HIV.

7. Utilisation selon la revendication 6, où le facteur de nécrose des tumeurs est le facteur-$\alpha$ de nécrose des tumeurs.

8. Utilisation selon la revendication 6 ou 7, où l'interféron est l'interféron $\alpha$, $\beta$ ou $\gamma$.

9. Utilisation selon la revendication 6, où l'interféron est l'interféron-$\gamma$.

10. Utilisation selon la revendication 6, où l'interféron est un mélange d'interféron gamma plus un interféron alpha ou bêta.

11. Utilisation selon l'une des revendications 6 à 9, en ce qui concerne l'infection par HIV.

12. Utilisation selon l'une des revendirations 6 à 10, où le médicament est pour une administration <u>ex vivo</u> en cours d'immunothérapie adoptive.

13. Utilisation selon l'une des revendications 6 à 11, où l'interféron doit être administré avant le facteur de nécrose des tumeurs.

14. Utilisation selon l'une des revendications 6 à 13, où les dosages du facteur de nécrose des tumeurs et d'interféron sont chacun d'environ 1 à 25 $\mu g/m^2/24$ heures par perfusion intraveineuse en continu.

15. Utilisation selon l'une des revendications 6 à 14, où le médicament comprend de plus un anticorps capable de neutraliser l'infection virale.

16. Utilisation selon la revendiaction 11, où l'administration d'un facteur de nécrose des tumeurs et d'interféron doit être répétée au cours d'environ 1 à 5 cycles.

17. Utilisation selon la revendication 11, où le médicament comprend des moyens pour immuniser l'animal contre le HIV avant l'administration d'un facteur de nécrose des tumeurs ou d'un facteur de nécrose des tumeurs et d'un interféron.

18. Utilisation selon l'une des revendications 6 à 17, où le médicament comprend de plus une substance inhibitrice à radicaux libres de l'oxygène physiologiquement acceptable.

19. Utilisation selon la revendication 18, où la substance inhibitrice est la catalase.

20. Utilisation selon la revendication 19, où la substance inhibitrice est la catalase d'érythrocyte humain.

21. Utilisation d'un facteur de nécrose des tumeurs, d'un interféron et d'une substance inhibitrice à radicaux libres de l'oxygène dans la fabrication d'un médicament.

22. Utilisation selon la revendication 21, où la substance inhibitrice est une enzyme active de manière peroxydante.

23. Utilisation selon la revendication 22, où la substance inhibitrice est la catalase.

24. Utilisation selon la revendication 23, où l'enzyme est une catalase d'érythrocyte humain.

25. Utilisation selon l'une des revendications 21 à 24, où le médicament comprend de plus une substance capable de prévenir (a) la réplication d'un rétrovirus ou (b) la liaison rétrovirale à un récepteur de surface cellulaire.

26. Utilisation d'un facteur de nécrose des tumeurs, d'un interféron et d'une substance capable de prévenir (a) la réplication d'un rétrovirus ou (b) la liaison rétrovirale à un récepteur de surface cellulaire dans la fabrication d'un médicament.

27. Utilisation selon la revendication 25 ou 26, où la substance capable de prévenir la liaison rétrovirale à un récepteur de surface cellulaire est un anticorps dirigé contre un polypeptide d'enveloppe du rétrovirus.

17

Fig.1. EMC/A549 Cells

Fig. 2. VSV/MDBK Cells

Fig.3.

IFN-γ Can Protect Against VSV Infection
in the Presence of TNF α/β

Fig.4a.

EP 0 235 906 B1

# Fig.4b.

Fig.5A.    EMC/A549

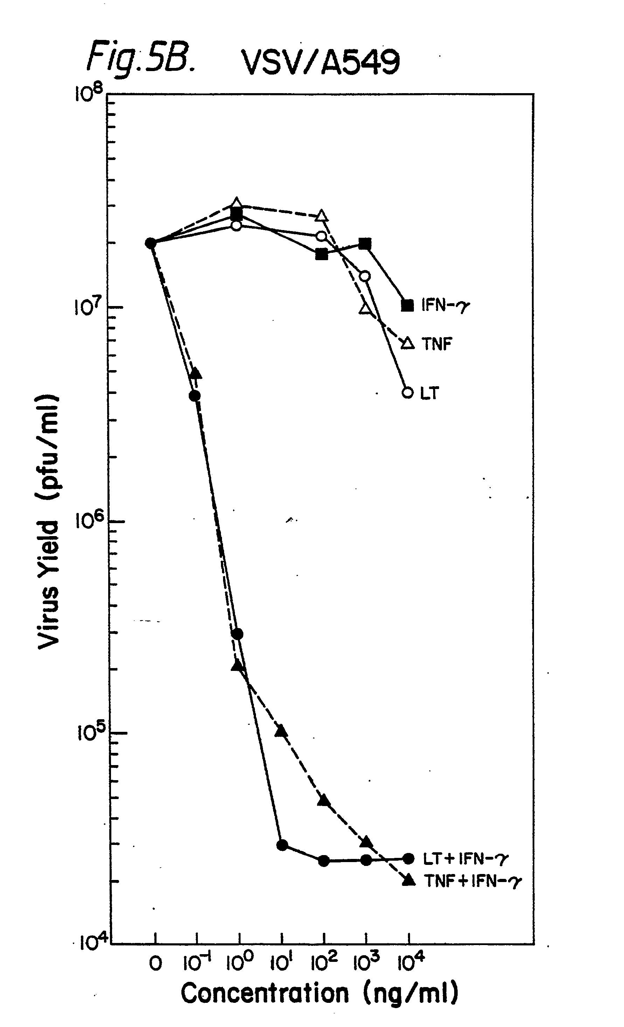

Fig.5B. VSV/A549

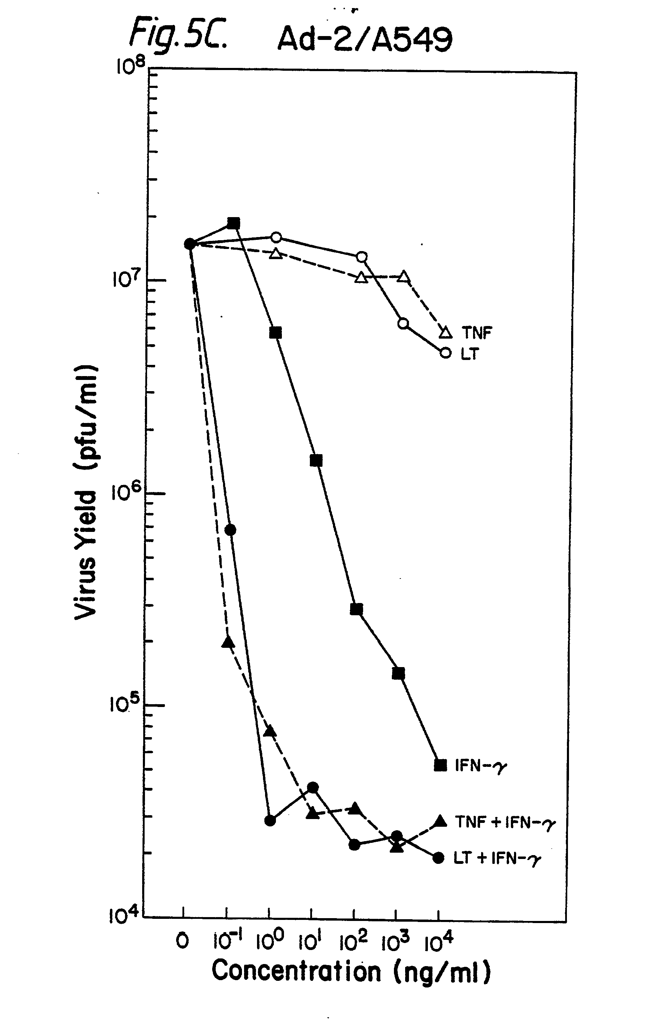

Fig.5C. Ad-2/A549

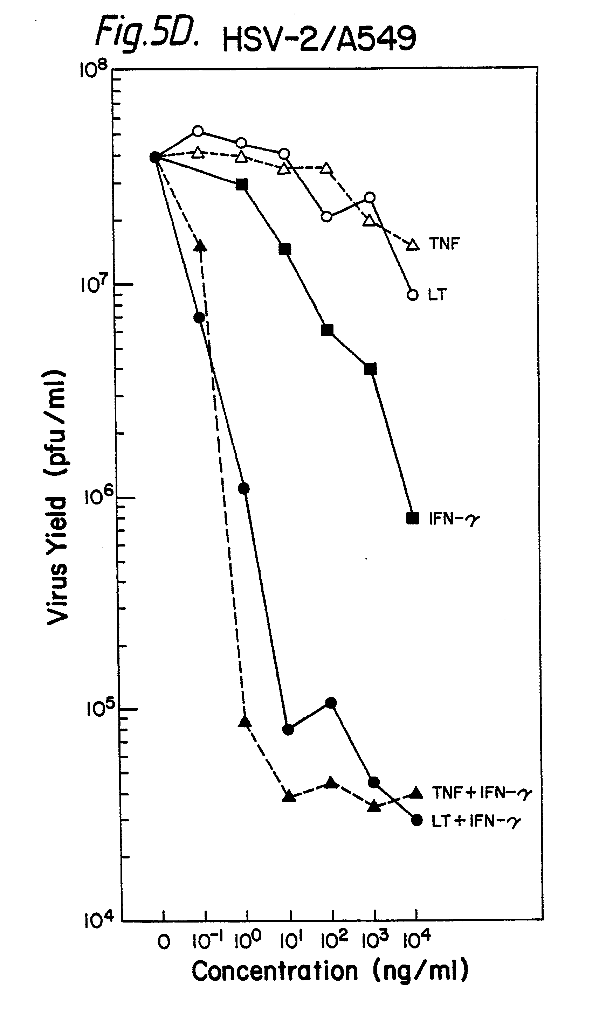

Fig.5D. HSV-2/A549

# Fig.6.

HIV

# Fig.7.

CATALASE enhances antiviral activity of IFN-ɣ & TNF